# EUROPEAN PATENT APPLICATION

(11) **EP 4 218 533 A1**
(43) Date of publication of application: **02.08.2023**
(21) Application number: 21918839.8
(22) Date of filing: 03.06.2021
(51) Int. Cl.: A61B 1/005

(54) **ENDOSCOPE HANDLE AND DRIVING DEVICE**

(30) Priority: 12.01.2021 CN 202110035277
(71) Applicant: Changzhou Lunghealth Medtech Company Limited, Jiangsu 213145 (CN)
(72) Inventor: WU, Haosheng, hangzhou, Jiangsu 213145 (CN); MA, Jiajun, hangzhou, Jiangsu 213145 (CN); ZANG, Jing, hangzhou, Jiangsu 213145 (CN)
(74) Representative: Renaudo, Adrien Hanouar
(86) International application number: PCT/CN2021/098154
(87) International publication number: WO 2022/151641

(57) **Abstract**

Disclosed are an endoscope handle and a driving apparatus (1). The driving apparatus (1) includes: a motor module (11), the motor module (11) being provided with a protruding motor shaft (111); a starting sliding block (13) connected to the motor shaft (111) and capable of being driven by the motor shaft (111) to move along a straight line, the starting sliding block (13) being provided with a first coupling portion (131), and the first coupling portion (131) being in coupling contact with a follow-up sliding block (21) externally disposed so as to drive, by means of the follow-up sliding block (21), a bending control wire (22) of an endoscope to be tightened and released; a force sensor (15) disposed on the motor module (11) and configured to measure a tensile force borne by the bending control wire (22) and generate a stress signal; and a controller communicatively connected to the motor module (11) and the force sensor (15) respectively and configured to adjust the output of the motor module (11) according to the stress signal fed back by the force sensor (15).

## Description

### CROSS-REFERENCE TO RELATED APPLICATIONS

The present disclosure refers to Chinese Patent Application No. 2021100352778, filed on January 12, 2021, and entitled "Endoscope Handle and Driving Apparatus", which is hereby incorporated by reference in its entirety.

### TECHNICAL FIELD

The present disclosure relates to the field of medical equipment, and in particular, to an endoscope handle and a driving apparatus.

### BACKGROUND

An endoscope is an apparatus that integrates an image sensor, an optical lens, an illumination source, and a precision mechanical structure. The endoscope may enter cavities of a human body via an oral cavity or other natural orifices and perform such operations as observation, biopsy and minimally invasive surgery on the tissues inside the human body. The endoscope in the related art allows a doctor to visually see a real-time internal picture of the human body, thereby improving convenience of diagnosis and treatment.

However, the endoscope in the related art relies entirely on the hand operation control of the doctor, and is prone to stab the delicate inner walls of the cavities in a case of improper operation.

### SUMMARY

In order to solve the above-mentioned problem or at least partially solve the above-mentioned technical problem, a driving apparatus for an endoscope handle is provided in an embodiment of the present disclosure, including:
a motor module, the motor module being provided with a protruding motor shaft;
a starting sliding block connected to the motor shaft and capable of being driven by the motor shaft to move along a straight line, the starting sliding block being provided with a first coupling portion, and the first coupling portion being in coupling contact with a follow-up sliding block externally disposed so as to drive, by means of the follow-up sliding block, a bending control wire of an endoscope to be tightened and released;
a force sensor disposed on the motor module and configured to measure a tensile force borne by the bending control wire and generate a stress signal; and
a controller communicatively connected to the motor module and the force sensor respectively and configured to adjust the output of the motor module according to the stress signal fed back by the force sensor.

Optionally, the driving apparatus further includes:
a housing, the motor module and the force sensor being both located inside the housing, a main portion of the starting sliding block being located inside the housing, and the first coupling portion protruding from a hole reserved on the housing.

Optionally, the motor module includes a motor body and a motor bracket, the motor body being connected to the housing by means of the motor bracket;
the force sensor is disposed on the motor bracket.

Optionally, the motor module includes a motor body directly and fixedly disposed on the housing, and the force sensor is disposed on a connection portion of the motor body and the housing.

Optionally, the driving apparatus further includes:
a position sensor disposed on the motor and configured to acquire number information of revolutions of the motor, the position sensor being communicatively connected to the controller; and
a stop switch communicatively connected to the controller and disposed on a motion path of the starting sliding block, the stop switch sending a stop signal to the controller when contacting the starting sliding block.

Optionally, the motor module includes at least three motors, a number of the starting sliding block is as many as and in one-to-one correspondence with the motors.

Optionally, the motor module includes four motors in one-to-one correspondence with four bending control wires, the four bending control wires being tightened and released to respectively control bending motions of the endoscope in four directions of a "cross".

Optionally, the driving apparatus further includes: a rocker communicatively connected to the controller and configured to control the tightening and releasing of the bending control wires by means of the four motors. A direction in which the rocker is pushed corresponds to a bending direction of the endoscope.

Another embodiment of the present disclosure provides an endoscope handle, including the foregoing driving apparatus and further including:
a driven apparatus detachably mounted on the driving apparatus, the driven apparatus including:
a follow-up sliding block, the follow-up sliding block being provided with a second coupling portion which is in coupling contact with the first coupling portion when the driven apparatus is mounted on the driving apparatus; and
a bending control wire connected to the follow-up sliding block so as to be tightened and released by the follow-up sliding block.

Optionally, the driving apparatus further includes:
a guide rail disposed in parallel to a motion direction of the starting sliding block, the follow-up sliding block being disposed on the guide rail.

Optionally, the first coupling portion protrudes towards the driven apparatus, the second coupling portion protrudes towards the driving apparatus, and a projection of the first coupling portion and a projection of the second coupling portion on a plane perpendicular to the motion direction of the follow-up sliding block at least partially overlap when the driven apparatus is mounted on the driving apparatus.

Optionally, in the presence of a plurality of follow-up sliding blocks, projections of connection lines of portions, connected to the bending control wires, of the respective adjacent follow-up sliding blocks on a plane perpendicular to the bending control wires constitute a regular polygon.

In contrast to the related art, embodiments of the present disclosure may monitor, by means of a force sensor, the force applied to a bending control wire when a catheter head of an endoscope is bent, thereby preventing the endoscope from injuring the walls of the cavities of a human body, and improving the safety and use comfort of the endoscope.

### BRIEF DESCRIPTION OF THE DRAWINGS

In order to more clearly illustrate the technical solutions in the embodiments of the present disclosure or in the related art, the drawings required to describe the embodiments or the related art will be briefly introduced below. It is obvious that the drawings in the following description are merely used for illustrating some embodiments of the present disclosure. Those ordinarily skilled in the art may also obtain the technical features, connection relationships and even method steps not mentioned in other drawings according to these drawings without involving any inventive effort.
FIG. 1 is a schematic stereogram of a driving apparatus for an endoscope handle according to an embodiment of the present disclosure;
FIG. 2 is a schematic stereogram of a driving apparatus for an endoscope handle when coupled to a driven apparatus according to an embodiment of the present disclosure;
FIG. 3 is a schematic diagram of a solution for disposing a force sensor of a driving apparatus for an endoscope handle according to an embodiment of the present disclosure;
FIG. 4 is a schematic diagram of another solution for disposing a force sensor of a driving apparatus for an endoscope handle according to an embodiment of the present disclosure;
FIG. 5 is a schematic diagram of still another solution for disposing a force sensor of a driving apparatus for an endoscope handle according to an embodiment of the present disclosure;
FIG. 6 is a modular block diagram of an endoscope handle in operation according to an embodiment of the present disclosure;
FIG. 7 is a schematic diagram of a combination relationship between a driving apparatus for an endoscope handle and a driven apparatus according to an embodiment of the present disclosure;
FIG. 8 is a schematic stereogram of a driven apparatus according to an embodiment of the present disclosure;
FIG. 9 is a schematic front diagram of a driving apparatus for an endoscope handle when coupled to a driven apparatus according to an embodiment of the present disclosure;
FIG. 10 is a schematic diagram of another follow-up sliding block adopted when a driving apparatus for an endoscope handle is coupled to a driven apparatus according to an embodiment of the present disclosure; and
FIG. 11 is a schematic diagram of still another follow-up sliding block adopted when a driving apparatus for an endoscope handle is coupled to a driven apparatus according to an embodiment of the present disclosure.

### Reference Numerals:

1, driving apparatus; 11, motor module; 111, motor shaft; 112, motor bracket; 12, housing; 13, starting sliding block; 131, first coupling portion; 14, position sensor; 15, force sensor; 16, stop switch; 2, driven apparatus; 21, follow-up sliding block; 211, second coupling portion; 22, bending control wire; 23, guide rail.

### DETAILED DESCRIPTION OF EXAMPLE EMBODIMENTS

For the purpose of making the objects, technical solutions and advantages of examples of the present disclosure clearer, the technical solutions in the examples of the present disclosure will now be described clearly and completely with reference to the drawings in the examples of the present disclosure. Obviously, the described examples are merely some, but not all, examples of the present disclosure. All other examples obtained by those ordinarily skilled in the art based on the examples in the present disclosure without involving creative efforts should fall within the protection scope of the present disclosure.

The applicants have found that an endoscope typically acts entirely according to the hand operation of doctors in the related art. The action of the endoscope is lack of force feedback, while a visual feedback displayed on a screen is two-dimensional. Therefore, when there is insufficient experience, it is easy to scratch or stab the inner walls of the cavities of a human body with a sharp front end of the endoscope due to excessive penetration.

In view of this, the present disclosure provides a novel endoscope handle and a driving apparatus.

### Embodiment 1

A first embodiment of the present disclosure provides a driving apparatus for an endoscope handle and an endoscope handle based on the driving apparatus.

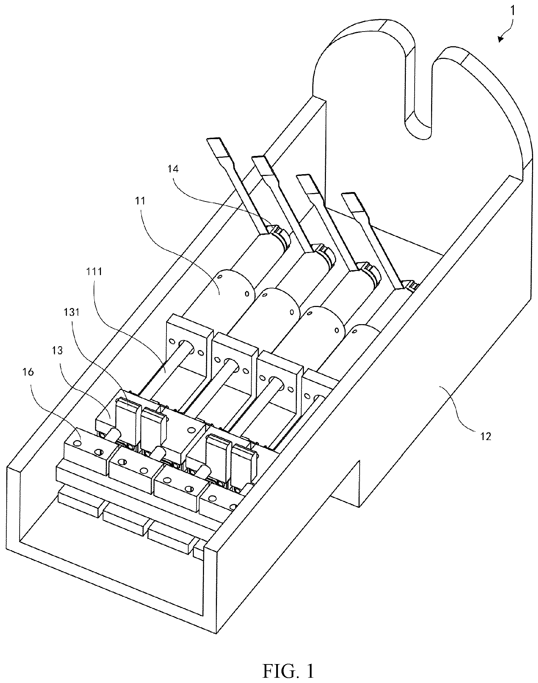

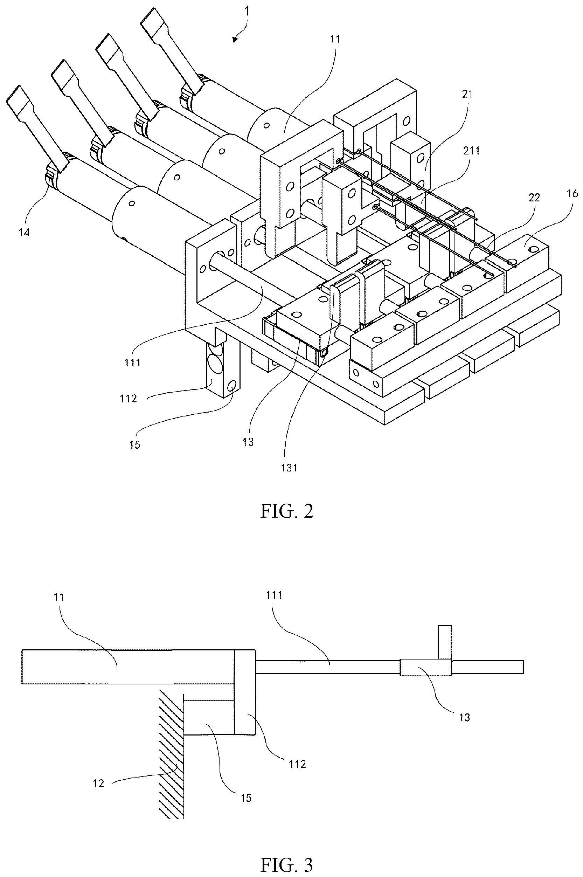

As shown in FIGs. 1 and 2, a driving apparatus 1 for an endoscope handle includes:
a motor module 11, the motor module 11 being provided with a protruding motor shaft 111;
a starting sliding block 13 being connected to the motor shaft 111 and capable of being driven by the motor shaft 111 to move along a straight line. The starting sliding block 13 is provided with a first coupling portion 131. The first coupling portion 131 is in coupling contact with a follow-up sliding block 21 externally disposed so as to drive, by means of the follow-up sliding block 21, a bending control wire 22 of an endoscope to be tightened and released. The tightening and releasing motion mentioned in the present disclosure refers to a motion of tightening or releasing the bending control wire 22. The motion, in conjunction with a multi-section structure of the endoscope, may bend the endoscope in a predetermined direction.

Specifically, the transmission connection between the starting sliding block 13 and the motor shaft 111 may be performed indirectly or directly. In the case of 1 shown in FIGs. 1 and 2, the starting sliding block 13 may be mounted directly on the motor shaft 111. When the starting sliding block 13 is provided with a thread, a lead screw structure may be formed with the motor shaft 111, so that the motor shaft 111 drives the starting sliding block 13 to move in a linear direction. That is, by means of the lead screw structure, it is realized that a rotational motion of the motor shaft 111 is converted into a linear motion of the starting sliding block 13. It should be understood in the art that other transmission manners capable of converting the rotational motion into the linear motion, such as a worm gear structure or a rack-and-pinion structure, can also substantially achieve the technical purpose of the present disclosure when applied between the starting sliding block 13 and the motor shaft 111.

The driving apparatus 1 may further include a housing 12. The housing 12 may be configured to protect and support an internal structure of the driving apparatus 1. The motor module 11 and the force sensor 15 are both located inside the housing 12. A main portion of the starting sliding block 13 is located inside the housing 12. When the housing 12 completely wraps the starting sliding block 13 therein, the first coupling portion 131 may protrude from a hole reserved on the housing 12, so as to ensure the coupling contact thereof with an external structure.

The coupling contact of the first coupling portion 131 with the follow-up sliding block 21 externally disposed aims to drive, by means of the follow-up sliding block 21, the bending control wire 22 of the endoscope to be tightened and released. Therefore, the coupling therebetween can be realized in various ways. For example, the first coupling portion 131 may be connected to the follow-up sliding block 21 externally disposed in addition to the contact manner illustrated in FIGs. 1 and 2. It should be particularly noted that the follow-up sliding block 21 is only moved by the starting sliding block 13, and therefore it is not a component of the "driving apparatus 1" for the endoscopic handle, but a part of a "driven apparatus 2" for the endoscopic handle.

As shown in FIG. 2, a force sensor 15 is additionally disposed in the present disclosure. The force sensor is disposed on the motor module 11, and capable of monitoring the force applied to the bending control wire 22 when a catheter head of the endoscope is bent, and generating a stress signal. Specifically, the force applied to the bending control wire 22 is transmitted in sequence through the follow-up sliding block 2, the starting sliding block 13 and a motor bracket 112, and eventually causes deformation of a sensing member of the force sensor 15. By measuring this deformation, the force applied to the bending control wire 22 may be obtained. The driving apparatus 1 is further provided with a controller communicatively connected to the motor module 11 and the force sensor 15 respectively and configured to adjust the output of the motor module 11 according to the stress signal fed back by the force sensor. The controller, as an electronic element, may be integrated at any position within the housing 12 or may be separately disposed outside the housing 12 for remote control, and therefore the position of the controller is not limited in the present disclosure and is not illustrated in the drawings.

It should be noted that the driving apparatus 1 may be further provided with a stop switch 16. The stop switch 16 is communicatively connected to the controller and disposed on a motion path of the starting sliding block 13. The stop switch 16 sends a stop signal to the controller when contacting the starting sliding block 13, indicating that the starting sliding block 13 has moved to the end of the stroke. Accordingly, the controller may stop the rotation of the motor after receiving the stop signal, thereby preventing the motor from being damaged by overload.

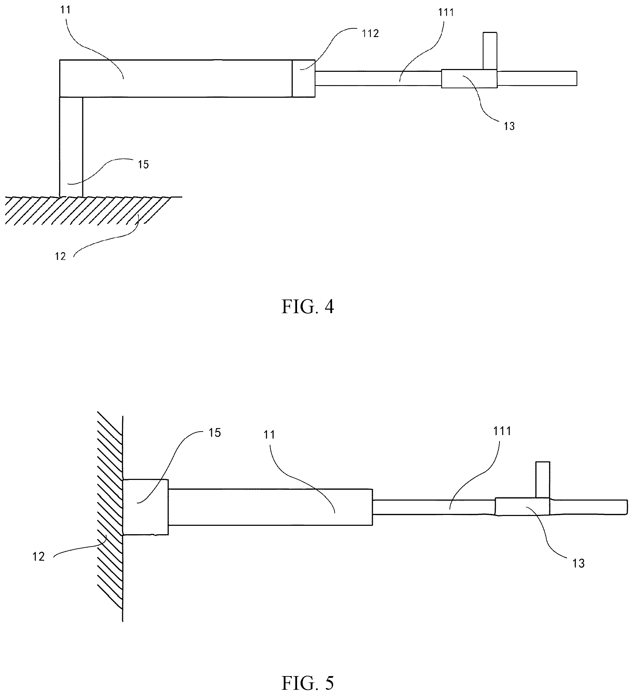

In the embodiments of the present disclosure, the force sensor 15 may be disposed in various ways. For example:
Optionally, as shown in FIGs. 3 and 4, the motor module 11 includes a motor body and a motor bracket 112. The motor body is mounted on the motor bracket 112. The motor bracket 112 is connected to the housing 12 by means of the force sensor 15.

Connection lines of the motor bracket 112, the force sensor 15 and the housing 12 in FIG. 3 are parallel to a motion direction of the starting sliding block 13, and connection lines of the motor bracket 112, the force sensor 15 and the housing 12 in FIG. 4 are perpendicular to the motion direction of the starting sliding block 13.

Or optionally, as shown in FIG. 5, the motor module 11 may include only a motor body directly connected to the housing 12 by means of the force sensor 15.

Connection lines of the motor bracket 112, the force sensor 15 and the housing 12 in FIG. 5 are parallel to the motion direction of the starting sliding block 13, and similarly, the connection lines may be perpendicular to the motion direction.

Since the effects of the forces are mutual, when the endoscope touches a human body, a part of the forces will be inevitably fed back to the bending control wire 22 and finally act on the motor module 11. The force sensor 15 may detect the deformation and transmit a corresponding deformation signal to the controller. Based on the above principle, by connecting the motor module 11 to the housing 12 through the force sensor 15, the controller may accurately obtain feedback force information so as to correspondingly modify and adjust the output of the motor module 11. For example, when it is detected that the motion of the endoscope is blocked, it may be determined that the endoscope has contacted an inner surface of the human body. At this moment, the endoscope may be retracted into a safe distance through feedback and compensation operations.

It should be noted that when the connection lines of the motor bracket 112, the force sensor 15 and the housing 12 are parallel to the motion direction of the starting sliding block 13, the feedback to the force sensor 15 is a positive pushing force or a positive pulling force. When the connection lines of the motor bracket 112, the force sensor 15 and the housing 12 are perpendicular to the motion direction of the starting sliding block 13, the feedback to the force sensor 15 is the stress in a shear direction.

Further optionally, the driving apparatus 1 may further include: a position sensor 14 disposed on the motor and configured to acquire number information of revolutions of the motor. The position sensor 14 is communicatively connected to the controller. A grating sensor may be selected as the position sensor 14. After the number information of revolutions acquired by the position sensor 14 is provided to the controller, the controller may accurately calculate a current specific position of the starting sliding block 13 accordingly, so as to provide an auxiliary reference for the compensation motion of the starting sliding block 13.

The motor module 11 may include at least two motors. Preferably, the motor module may include three or more motors. For example, as shown in FIGs. 1 and 2, the motor module may include four motors. When there are two motors, a lens end of the endoscope may move freely in one degree of freedom, which achieves the "linear" motion of the endoscope.

However, when there are three or more motors, four motors are in one-to-one correspondence with three bending control wires 22, and respectively control bending motions of the endoscope in three directions of a "herringbone" by means of the tightening and releasing actions. When two adjacent bending control wires 22 are tightened and released simultaneously, an inclined motion of the endoscope may be controlled. In other words, by combining the tightening and releasing actions of each bending control wire 22, any motion of 360° at the lens end of the endoscope can be realized, and an inclined angle of the endoscope can also be conveniently controlled by controlling the tightening and releasing degree of the two bending control wires 22.

In the case of four motors, the four motors are in one-to-one correspondence with four bending control wires 22, and the four bending control wires 22 may be tightened and released to respectively control bending motions of the endoscope in four directions of a "cross". Similarly, when two adjacent bending control wires 22 are tightened and released simultaneously, an inclined motion of the endoscope may be controlled. In other words, by combining the tightening and releasing actions of each bending control wire 22, any motion of 360° at the lens end of the endoscope can also be realized, an inclined angle of the endoscope can also be controlled by controlling the tightening and releasing degree of the two bending control wires 22, and the precision of the motion is higher than that in the case of three motors.

In the present disclosure, a number of the starting sliding block 13 is as many as and in one-to-one correspondence with the motors. Other structures including the bending control wire 22, the follow-up sliding block 21, the force sensor 15, the stop switch 16, and the position sensor 14 may also be disposed in a one-to-one correspondence. In other words, the controller may perform motion control and feedback compensation separately for each motor.

Based on the above solution, optionally, the driving apparatus 1 may further include: a rocker communicatively connected to the controller and configured to control the tightening and releasing of the bending control wires 22 by means of the four motors. A direction in which the rocker is pushed corresponds to a bending direction of the endoscope. It should be noted that since a remote sensor may be fixedly mounted at any position of the housing or connected to the controller through a wired or wireless scheme, a specific mounting structure of the remote sensor is not illustrated in the drawings.

The rocker is used to control the motion of the endoscope so as to achieve the operation logic that what you see is what you get, thereby reducing the control difficulty of the endoscope, and allowing a doctor to concentrate more on medical operations.

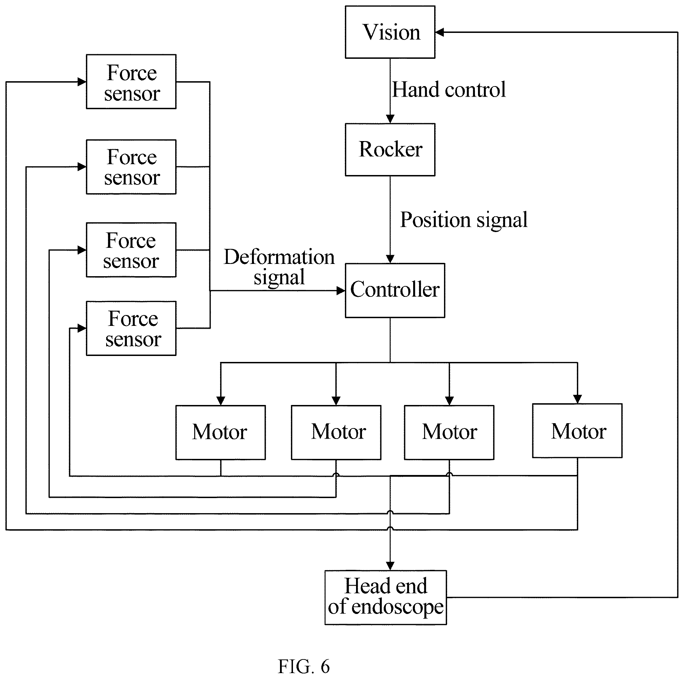

Based on the above structure, as shown in FIG. 6, an embodiment of the present disclosure further provides operation steps of the driving apparatus 1 as follows:
1. Depending on a picture fed back by the head end of the endoscope, a user may issue control commands to the controller by means of the rocker so that the controller drives various different motors respectively to move within the cavities of a human body.
2. Meanwhile, the pressure sensor 15 corresponding to each motor continuously feeds back a stress signal to the controller, and the controller may perform feedback compensation on the driving of the motor according to these chip signals.
3. In addition, the position sensor 14, the stop switch 16 and other mechanisms can further assist the motor in correcting the position of the starting sliding block 13.

In summary, in contrast to the related art, the embodiments of the present disclosure can effectively prevent, by means of the force sensor 15, the endoscope from injuring the human body due to excessive penetration, thereby improving the safety and use comfort of the endoscope.

### Embodiment 2

The inventors of the present disclosure have found that most endoscopes of the related art are reusable due to cost limitations. Therefore, the endoscope needs to be thoroughly disinfected and cleaned after use for each patient. The cleaning process is cumbersome and has a negative impact on the efficiency of diagnosis and treatment.

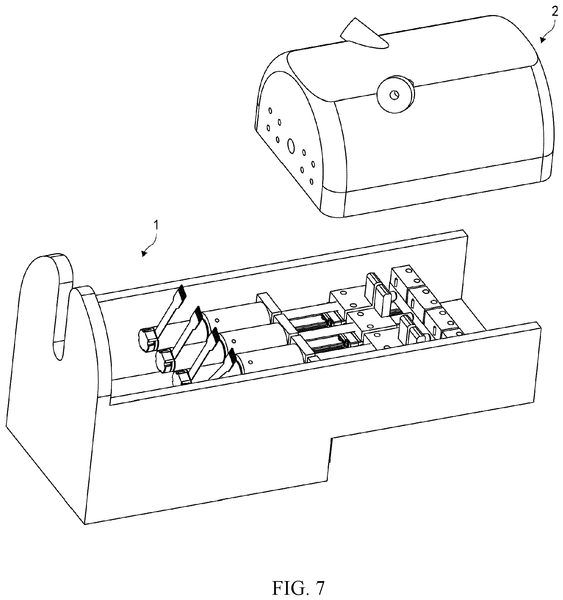

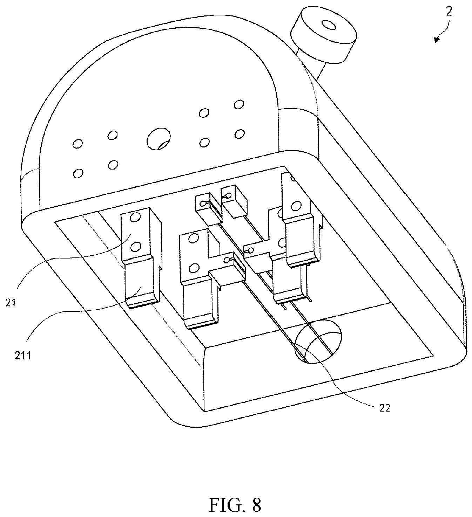

In view of this, a second embodiment of the present disclosure provides an endoscopic handle. As shown in FIG. 7, the endoscopic handle, in addition to the driving apparatus 1 mentioned in the first embodiment, also includes:
a driven apparatus 2 detachably mounted on the driving apparatus 1. As shown in FIG. 8, the driven apparatus 2 may include a follow-up sliding block 21. The follow-up sliding block 21 is provided with a second coupling portion 211. The second coupling portion 211 is in coupling contact with the first coupling portion 131 when the driven apparatus 2 is mounted on the driving apparatus 1.

A bending control wire 22 is connected to the follow-up sliding block 21 so as to be tightened and released by the follow-up sliding block 21.

In addition, other components closely associated with entry of the cavities of the human body, including suction pipelines, spray pipelines, clamp pipelines, etc., may be disposed on the driven apparatus 2. Since the driven apparatus 2 is detachable, the driven apparatus in the endoscope after each use may be discarded and replaced with a new driven apparatus 2, so that consumables contacting the body part of a patient are disposable, the convenience can be improved while the risk of cross-contamination is prevented, and the safety is improved.

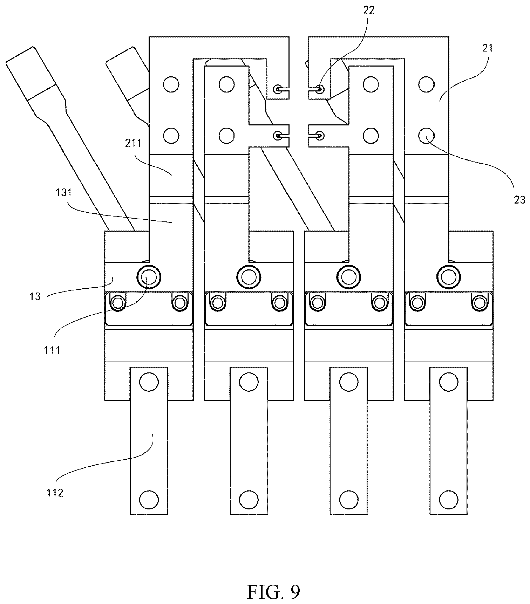

In addition to the above structure, as shown in FIG. 9, the driven apparatus 2 may further include a guide rail 23 disposed in parallel to a motion direction of the starting sliding block 13. The follow-up sliding block 21 is disposed on the guide rail 23. By means of the guide rail 23, the motion track of the follow-up sliding block 21 can be accurately controlled, and the running stability of the equipment can be improved.

The present disclosure does not specifically limit the coupling relationship between the first coupling portion 131 and the second coupling portion 211. It is only necessary to ensure that the starting sliding block 13 drives the follow-up sliding block 21 to move within a satisfactory range of travel.

In other words, optionally, as shown in FIG. 9, the first coupling portion 131 may protrude towards the driven apparatus 2, the second coupling portion 211 protrudes towards the driving apparatus 1, and a projection of the first coupling portion 131 and a projection of the second coupling portion 211 on a plane perpendicular to the motion direction of the follow-up sliding block 21 at least partially overlap when the driven apparatus 2 is mounted on the driving apparatus 1.

The projection overlap may ensure that the first coupling portion 131 can touch and push the second coupling portion 211 to move during the motion. This projection overlap may be achieved in various ways. For example, the first coupling portion 131 and the second coupling portion 211 may be coupled. For example, a slot may be provided on the first coupling portion 131, and the second coupling portion 211 may be inserted into the slot of the first coupling portion 131. For another example, the coupling portions may be connected by clamping, screwing or the like.

Furthermore, the coupling between the sliding blocks may also take the form of non-connection, while the force is transmitted only by touching and pushing. For example, the first coupling portion 131 is located at a position near the distal end, and the second coupling portion 211 is located at a position near the proximal end. When the rocker is pushed, the controller controls the motor to rotate so as to drive the starting sliding block 13 to move along a straight line. At this moment, the second coupling portion 211 pushes the first coupling portion 131 to move, so that the bending control wire 22 is tightened. On the contrary, when the rocker is released, the controller controls the motor to rotate reversely, and also drives the starting sliding block 13 to return. At this moment, the first coupling portion 131 is not in contact with the second coupling portion 211, the bending control wire 22 may automatically spring back, and the endoscope returns to an equilibrium position.

This structure has the advantage of being simpler than the connection structure, and when the apparatus returns to an equilibrium position by automatically springing back, the damage caused by excessive travel of the reverse motion can be prevented.

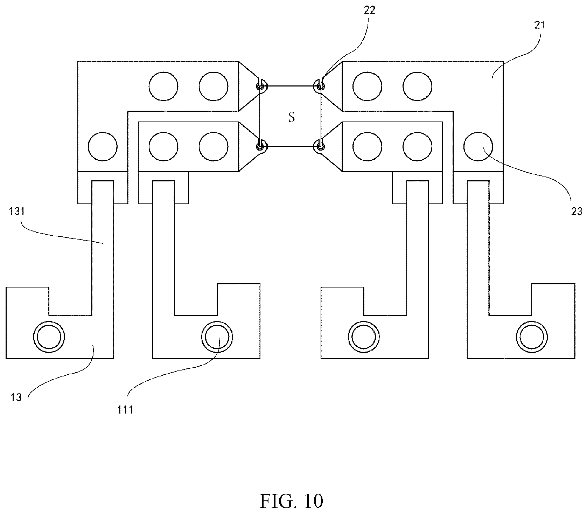

In addition, referring to FIGs. 9, 10 and 11, in the presence of a plurality of follow-up sliding blocks 21, projections of connection lines of portions, connected to the bending control wires 22, of the respective adjacent follow-up sliding blocks 21 on a plane perpendicular to the bending control wires 22 constitute a regular polygon. In the figures, since there are four bending control wires 22, a regular quadrangle, such as square S in FIG. 10, is constituted.

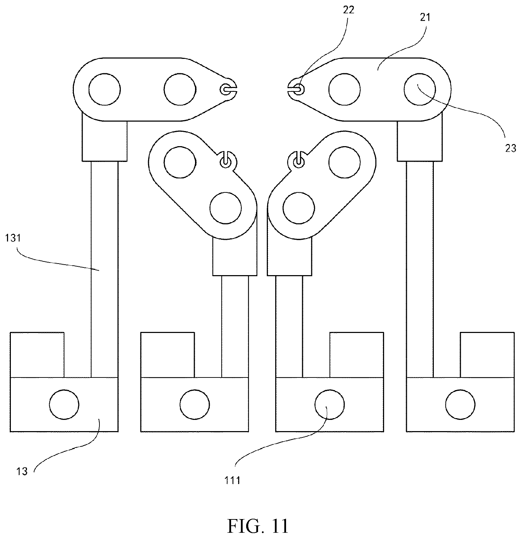

In contrast to being arranged in a straight line, the portions connected to the bending control wires 22 are surrounded, so that the bending control wires 22 can be more uniformly stressed, thereby improving the reliability. It should be noted that follow-up sliding blocks 21 of various shapes may be adopted in order to achieve this surrounding layout. As shown in FIG. 9, there are two follow-up sliding blocks 21 wrapping the other two follow-up sliding blocks; or as shown in FIG. 10, the follow-up sliding blocks are arranged in two parallel rows in a height direction; or as shown in FIG. 11, the portions connected to the bending control wires 22 are disposed at different positions of different follow-up sliding blocks. The above-mentioned arrangements all achieve the purpose of uniformly stressing the bending control wires 22.

It should be understood that the terms used in the examples of the present disclosure is for the purpose of describing particular examples only, and is not intended to limit the present disclosure. As used in the examples of the present disclosure and the appended claims, the singular forms "a/an", "the", and "this" are intended to include the plural forms as well, unless the context clearly indicates otherwise, "plural" generally means at least two, but may also mean at least one.

It should be understood that the term "and/or" used herein describes only an association relationship for describing associated objects and represents that three relationships may exist. For example, a first member and/or a second member may represent the following three cases: only the first member exists, both the first member and the second member exist, and only the second member exists. In addition, the character "/" herein generally represents an "or" relationship between the associated objects.

It should be understood that although the terms first, second, third, etc. may be used in the examples of the present disclosure to describe certain members, these members should not be limited to only these terms. These terms are only used to distinguish one member from another. For example, a first certain member may also be referred to as a second certain member, and similarly, a second certain member may also be referred to as a first certain member, without departing from the scope of the examples of the present disclosure.

Depending on the context, the words "if" and "supposing", as used herein, may be interpreted as "during" or "when" or "in response to determining" or "in response to monitoring". Similarly, depending on the context, the phrase "if determined" or "if monitored (stated condition or event)" may be interpreted as "when determined" or "in response to determining" or "when monitored (stated condition or event)" or "in response to monitoring (stated condition or event)".

In the embodiments of the present disclosure, the terms "substantially equal to", "substantially perpendicular to", "substantially symmetrical" and the like mean that the macroscopic sizes or relative positional relationship between two features referred to is very close to the "and" relationship. However, it will be clear to those skilled in the art that due to the existence of objective factors such as errors and tolerances, the positional relationship of objects is difficult to be exactly constrained at a small scale or even at a microscopic angle. Therefore, even if there is a slight error in the size and positional relationship therebetween, the achievement of the technical effect of the present disclosure will not be greatly affected.

It should also be noted that the terms "including", "containing" or any other variations thereof are intended to cover a non-exclusive inclusion, such that an article or system including a series of elements not only includes those elements, but also includes other elements that are not explicitly listed, or also includes elements inherent to such article or system. It is not excluded, without more constraints, that additional identical elements exist in the article or system including elements defined by the statement "including a ...".

In the various embodiments described above, while the methods are illustrated and described as a series of actions for simplicity of explanation, those ordinarily skilled in the art will understand and appreciate that the methods are not limited by the order of actions, as some actions may, in accordance with one or more examples, occur in different orders and/or concurrently with other actions which are illustrated and described herein or not illustrated and described herein but will be understood by those ordinarily skilled in the art.

Those skilled in the art will understand that information, signals and data may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Those skilled in the art will further appreciate that the various illustrative logical blocks, modules, units, circuits, and algorithm steps described in conjunction with the examples disclosed herein may be implemented as electronic hardware, computer software, or a combination of both. To clearly illustrate this interchangeability of hardware and software, various illustrative components, blocks, modules, units, circuits, and steps have been described above generally in terms of their functionality. Whether such functionality is implemented as hardware or software depends upon the particular application and design constraints imposed on the overall system. Those skilled in the art may implement the described functionality in varying ways for each particular application, but such implementation decisions should not be interpreted as causing a departure from the scope of the present disclosure.

Finally, it should be noted that those ordinarily skilled in the art may understand that the embodiments of the present disclosure set forth numerous technical details for the reader to better understand the present disclosure. However, the technical solutions claimed in the claims of the present disclosure may be basically realized without these technical details and various changes and modifications based on the above-mentioned various embodiments. Accordingly, various changes in form and detail may be made in the above-described embodiments in practical applications without departing from the spirit and scope of the present disclosure.

## Claims

1. A driving apparatus for an endoscope handle, comprising:
a motor module, the motor module being provided with a protruding motor shaft;
a starting sliding block connected to the motor shaft and capable of being driven by the motor shaft to move along a straight line, the starting sliding block being provided with a first coupling portion, and the first coupling portion being in coupling contact with a follow-up sliding block externally disposed so as to drive, by means of the follow-up sliding block, a bending control wire of an endoscope to be tightened and released;
a force sensor disposed on the motor module and configured to measure a tensile force borne by the bending control wire and generate a stress signal; and
a controller communicatively connected to the motor module and the force sensor respectively and configured to adjust the output of the motor module according to the stress signal fed back by the force sensor.

2. The driving apparatus for the endoscope handle according to claim 1, further comprising:
a housing, the motor module and the force sensor being both located inside the housing, wherein a main portion of the starting sliding block is located inside the housing, and the first coupling portion protrudes from a hole reserved on the housing.

3. The driving apparatus for the endoscope handle according to claim 2, wherein the motor module comprises a motor body and a motor bracket, the motor body being connected to the housing by means of the motor bracket;
the force sensor is disposed on the motor bracket.

4. The driving apparatus for the endoscope handle according to claim 2, wherein the motor module comprises a motor body, the motor body is directly and fixedly disposed on the housing, and the force sensor is disposed on a connection portion of the motor body and the housing.

5. The driving apparatus for the endoscope handle according to claim 1, further comprising:
a position sensor disposed on the motor and configured to acquire number information of revolutions of the motor, the position sensor being communicatively connected to the controller; and
a stop switch communicatively connected to the controller and disposed on a motion path of the starting sliding block, the stop switch sending a stop signal to the controller when contacting the starting sliding block.

6. The driving apparatus for the endoscope handle according to any one of claims 1 to 5, wherein the motor module comprises at least three motors, a number of the starting sliding block is as many as and in one-to-one correspondence with the motors.

7. The driving apparatus for the endoscope handle according to claim 6, wherein the motor module comprises four motors in one-to-one correspondence with four bending control wires, the four bending control wires being tightened and released to respectively control bending motions of the endoscope in four directions of a "cross".

8. The driving apparatus for the endoscope handle according to claim 7, further comprising: a rocker communicatively connected to the controller and configured to control the tightening and releasing of the bending control wires by means of the four motors, wherein a direction in which the rocker is pushed corresponds to a bending direction of the endoscope.

9. An endoscope handle, comprising the driving apparatus according to any one of claims 1 to 8, further comprising:
a driven apparatus detachably mounted on the driving apparatus, the driven apparatus comprising:
a follow-up sliding block, the follow-up sliding block being provided with a second coupling portion, wherein the second coupling portion is in coupling contact with the first coupling portion when the driven apparatus is mounted on the driving apparatus; and
a bending control wire connected to the follow-up sliding block so as to be tightened and released by the follow-up sliding block.

10. The endoscope handle according to claim 9, further comprising:
a guide rail disposed in parallel to a motion direction of the starting sliding block, the follow-up sliding block being disposed on the guide rail.

11. The endoscope handle according to claim 9, wherein the first coupling portion protrudes towards the driven apparatus, the second coupling portion protrudes towards the driving apparatus, and a projection of the first coupling portion and a projection of the second coupling portion on a plane perpendicular to the motion direction of the follow-up sliding block at least partially overlap when the driven apparatus is mounted on the driving apparatus.

12. The endoscope handle according to claim 9, wherein in the presence of a plurality of follow-up sliding blocks, projections of connection lines of portions, connected to the bending control wires, of the respective adjacent follow-up sliding blocks on a plane perpendicular to the bending control wires constitute a regular polygon.
